# EUROPEAN PATENT SPECIFICATION

(11) **EP 3 181 104 B1**
(45) Date of publication and mention of the grant of the patent: **22.01.2020**
(21) Application number: 15199989.3
(22) Date of filing: 15.12.2015
(51) Int. Cl.: A61F 13/02, A61F 13/00

(54) **CATHETER SECUREMENT DRESSING**
KATHETERSICHERUNGSVERBAND
PANSEMENT DE FIXATION DE CATHÉTER

(43) Date of publication of application: 21.06.2017
(73) Proprietor: 3M Innovative Properties Company, St. Paul, MN 55133-3427 (US)
(72) Inventor: Graichen, Andreas Heinz, 40721 Hilden (DE); Palka-Santini, Maria Elisabete, 50585 Köln (DE); Burkard, Mariana Rosa Yzusqui, 78628 Rottweil (DE); Kozyra, Stephanie, 93173 Wenzenbach (DE)
(74) Representative: Gabriel, Kiroubagaranne

(56) References cited:
- WO-A1-2009/143913
- DE-U1- 29 503 926
- DE-U1-202015 001 021
- US-A1- 2004 143 220
- US-A1- 2006 002 988
- US-A1- 2012 150 122

## Description

The invention relates to medical dressings for catheter securement. The invention relates in particular to self-adhesive catheter securement dressings.

Protection of the site of insertion of vascular catheters often requires application of a medical dressing over the catheter insertion site to prevent soiling or contamination of the breached skin. Medical dressings generally reduce exposure of the skin to bacteria and to mechanical stimuli. Such medical dressings, for covering of catheter insertion points, are almost always factory-made, prefabricated, industrial products of mostly rectangular, oval or circular shape. Many dressings are self-adhesive in that they comprise a layer of adhesive which can stick to the skin and in use affixes the dressing to the patient's skin. In order to provide sufficient adhesion to the skin so that the catheter is securely held in place, most catheter securement dressings are comparatively large, e.g. 10cm long and 5cm wide.

An example of a catheter securement dressing is mentioned in the U.S. patent application US 2004/0143220 A1. It relates to a film dressing for intravenous catheter sites with at least two adhesive layers, in which additional strips may be used to secure the dressing.

Self-adhesive dressings tend to come off the skin over time, mostly due to mechanical forces. Adhesion to skin is reduced quickly at body joints where the skin is repeatedly pulled tight and pushed together forming folds as the patient moves the joint. Many dressings cannot follow this movement of the skin for long time, and a part of the dressing comes off in the area of the joint.

For example, it is difficult to affix a large dressing to the lateral neck area of a human patient, say for securement of a jugular catheter, such that it stays on for a longer time. This is because a part of a large dressing may extend beyond the lateral neck onto the skin of the lower jaw, and another part may extend beyond the lateral neck onto the skin around the collar bone. When the patient moves his jaw, wrinkles may form in the area where the jaw and the lateral neck meet, and when the patient turns or bends his head, wrinkles form in the area where the collar bone area and the neck meet. In these areas self-adhesive dressings are likely to detach from the skin after a few hours, depending on the frequency and amplitude of the patient's movement. Also, where a dressing extends beyond a joint into an adjacent body area, the patient may be impeded in his normal movement of the joint, and may feel uncomfortable as the dressing pulls on his skin when he moves the joint.

Once a first part of the dressing has come off the skin, other portions will follow quickly. A dressing that does not adhere properly cannot protect the skin against ingress of bacteria sufficiently and needs to be changed. However, changing a medical dressing is potentially painful for the patient, increases the risk of infections, may cause dysfunction of the catheter used, and requires a health care worker's labour time.

It is thus desirable to provide self-adhesive catheter securement dressings that stay affixed to the skin for longer time and need changing less frequently. In particular, it would be desirable to provide pre-fabricated dressings having these properties. More particularly, it is desirable to provide larger, pre-fabricated self-adhesive dressings for the lateral neck area of human patients that stay affixed to the skin for longer time. Also, it is desirable to provide larger dressings that are more comfortable to wear and cause less discomfort.

The present invention attempts to address these needs.

The present invention provides a pre-fabricated self-adhesive flat catheter securement dressing for application to a lateral neck area of a human patient, comprising a layer of pressure-sensitive adhesive for affixing the dressing to the patient's skin, and comprising a window for facilitating visual inspection of the patient's skin, characterized in that the dressing, in the plane of the dressing, has an elongated, generally triangular outer shape defined by a first long edge, a second long edge opposite to the first long edge, and at least one short edge connecting an end portion of the first long edge and an end portion of the second long edge, and wherein the triangular outer shape has no axial symmetry.

The triangular shape of the inventive dressing provides that the dressing does not extend beyond the lateral neck area. In particular, it provides that the dressing does not extend over joints into adjacent body parts. Avoiding extension over joints reduces significantly the risk of a part of the dressing coming off the skin in these joint areas, with the associated risk of bacteria ingress. On the other hand, the inventive dressing is large enough to provide a substantial adhesive contact surface for contact to the skin. This large surface facilitates more reliable securement of a catheter and, potentially, of lumen leading towards the catheter. Even a larger and heavier catheter can thereby be secured to the skin. The large contact surface affixes the catheter more securely to the skin. The patient can therefore perform certain movements of his neck with less risk of the dressing coming off the skin and the catheter not being held securely any more. The anatomic shape of the dressing fits the shape of the lateral neck and makes good use of the available skin surface of the lateral neck area for attachment of the dressing.

A catheter securement dressing generally differs from other dressings in that it comprises dedicated means for securing a catheter on the skin of a patient or means to secure lumen, leading towards or from the catheter, on the skin of a patient. These means can be, for example, slits or cuts or recesses, protrusions, openings, adhesive-coated surfaces, layers of adhesive, or integrated or separate securement strips.

The inventive dressing is a self-adhesive dressing. It comprises a layer of adhesive for affixing the dressing to the patient's skin. The adhesive is a pressure-sensitive adhesive ("PSA"). The layer of adhesive may be continuous. Alternatively, the layer of adhesive may be patterned, i.e. the layer may either comprise a plurality of adhesive portions, separated by non-adhesive portions, or it may comprise a plurality of non-adhesive portions, separated by adhesive portions.

The dressing according to the invention comprises a window portion for facilitating inspection of the patient's skin underneath the dressing. A layer of adhesive may extend into the window portion or completely cover the window portion. A window portion may be formed, for example, by an opening in a layer or in all layers of a dressing. A window portion may be formed, for example, by a transparent portion of one or more layers of a dressing.

Catheter securement dressings according to the invention are prefabricated. In other words, these dressings are industrially made in larger quantities (e.g. tens or hundreds) in identical shape. This is to delimit the inventive dressing from any dressings individually cut to shape, e.g. by hand.

A dressing according to the invention is thin, i.e. the dressing has two opposed major surfaces extending in respective width and length directions. The extensions of a thin dressing in the width and length directions are much larger, for example more than ten times larger or more than hundred times larger, than its extension in a thickness direction.

The dressing of the present invention is flat, i.e. it is an essentially two-dimensional body, before it is applied. This is to say that the dressing may not have a major surface curved in two directions before it is applied. In other words, the inventive dressing may be flattened out on a flat, non-curved surface without forming wrinkles. When a thin dressing is flattened out, it defines a geometric plane, this plane may be called "the plane of the dressing".

A catheter securement dressing according to the present invention has an elongated, generally triangular outer shape. This refers to the dressing before being applied to the patient. "Elongated" and "triangular" are standard terms, and should be read as having their normal meanings. "Elongated" is sometimes interpreted as to indicate an aspect ratio of length/width which is considerably larger than 1, like for example 1.2 or 1.5. In the present disclosure, an aspect ratio of 1.2 or larger defines an elongated shape.

The term "triangular" generally includes many "diverging" shapes, like for example isosceles triangles, scalene triangles, right-angled triangles, or obtuse triangles. In the present disclosure, the definition of "triangular" is more directed to the number of sides or edges of the dressing rather than to the clear visibility of the angles. This is because many of the dressings according to the present disclosure have rounded corners in order to improve adhesion to skin, so that the angles may not be visible. A dressing in the shape of a triangle with rounded corners is still considered a dressing of generally triangular shape.

In the present disclosure, the term "triangular" includes generally triangular shapes with one, two or three curved edges. Such curved edges of a triangle may be curved inwardly, i.e. recessed towards the interior of the triangle, or outwardly, i.e. protruding from the interior of the triangle.

An elongated, generally triangular outer shape thus includes triangular shapes in which the triangle has two long edges and one shorter edge. The long edges are located generally opposite to each other. The long edges are not parallel to each other. The short edge may be at any angle with one of the long edges, for example at an acute angle, at a 90-degree or right angle, or at an obtuse angle.

Alternatively to an "elongated, triangular" dressing, the outer shape of a dressing according to the invention can also be described as having an elongated, diverging shape defined by a first long edge, a second long edge opposite to the first long edge, and at least one short edge connecting an end portion of the first long edge and an end portion of the second long edge. The diverging shape stems from the long edges not being parallel.

A dressing according to the invention having an elongated diverging shape may be a trapezoidally-shaped dressing.

A dressing according to the invention having an elongated diverging shape may comprise a second short edge, connecting the end portion of the first long edge and the end portion of the second long edge that are not connected by the first short edge.

The dressing according to the present invention is shaped such as to fit on the lateral neck area of a human patient. The lateral neck area comprises the area below the ear and the area of the neck below the cheek. In particular, it comprises the area which is typically used to attach catheter securement dressings to, in particular those for securing jugular catheters. Human anatomy is such that the vertical extension of the lateral neck area (i.e. in vertical direction when the patient stands with his head upright) is generally smaller towards the throat and generally larger towards the back of the head or the spine. Hence the inventive dressing is preferably to be applied with the first long edge arranged parallel to the jaw edge of the patient, such that the narrower portion of the elongated, triangular dressing is arranged towards the throat and the wider portion, adjacent to the short edge, is arranged towards the back.

The outer shape of the dressing may be defined by a contour edge in the shape of a smooth closed curve, which is differentiable at any point of the curve. The expression "differentiate" is to be read as a mathematical term. A curve that is differentiable anywhere is a curve that has no corners, steps or sharp vertices, in other words a smooth curve. A self-adhesive dressing that has a smooth contour without corners or vertices is likely to stick better and longer to skin, because self-adhesive dressings tend to come off the skin first in the area of corners or vertices.

The outer shape of a dressing according to the invention may have an aspect ratio, defined as the greatest length of the dressing in any direction divided by the greatest width in a direction perpendicular to the direction of the greatest length, of 1.5 or more. Dressings of such aspect ratios fit particularly well to the lateral neck area of a human patient, in particular if the greatest width of the dressing is chosen to be slightly smaller than the vertical extension of the lateral neck between jaw area and shoulder area.

Generally a dressing according to the invention may have a greatest width as defined above that is 10%, 20% or 30% smaller than the vertical extension of the lateral neck between jaw area and shoulder area. A dressing according to the invention may have a greatest width as define above, that is 10%, 20% or 30% smaller than the vertical extension of the lateral neck between skull bone at the ear and shoulder area. Self-adhesive dressings with such greatest widths may fit particularly well to the lateral neck area and may provide a particularly large adhesive contact surface for contact to the skin.

In a dressing according to the invention, the first long edge may define a curved line, the curved line being shaped such that the dressing comprises, in the plane of the dressing, a concave portion being concave to the outside of the dressing. The first long edge may comprise, in the plane of the dressing, a portion concave to the outside of the dressing. A concave long edge may make the dressing fit particularly well to the lateral neck area of a patient, so that the dressing does not extend into other body areas, e.g. over joints. The concave portion of the dressing may be particularly suitable to be applied around the jaw corner.

The expression "concave" refers to the outer shape of the dressing when the dressing is flat. A concave portion may be formed by the long edge defining an inwardly curved or an inwardly angled portion, like for example a crescent moon shape or the shape of the letter C. The degree of curvature may be much less than the curvature in the letter C. The concave portion may be formed by the long edge defining an inwardly curved or an inwardly angled portion, like for example in the bracket character ")".

The concave portion is concave to the outside of the dressing. Thus when the dressing is flat, the concave portion of the dressing can be described as a recess in the dressing, lying in the plane of the dressing.

In a dressing according to the invention, the first long edge may define a first curved line, disregarding corner portions of the first long edge. The second long edge may define a second curved line, disregarding corner portions of the second long edge. The first curved line and the second curved line may be curved in the same direction. The centre of curvature for the first curved line and the centre of curvature of the second curved line may be on the same side of the first curved line, when viewed from a direction normal to the plane of the dressing, when the dressing is flattened out or flat..

Dressings of a shape so defined may resemble again a crescent moon. The curved long edges provide for a particularly good fit to the lateral neck area of many patients, since this area is limited by similarly curved lines.

The outer shape of dressing may comprise a corner, wherein the corner is rounded at a radius of 1 cm or more. As stated above, a rounded corner may provide better adhesion, greater adhesion reliability and less risk of adhesive failure than a non-rounded, sharp corner. Generally, the larger the radius of a rounded corner, the better the adhesion, the greater the reliability, and the smaller the risk of the dressing coming off the skin in the area of the corner. A rounding radius of 1cm or more provides a reduced risk of adhesive failure.

In a particular aspect of the invention, the outer shape of the dressing may comprise three corners, wherein each corner is rounded. Such a dressing provides particularly good adhesion to skin at the corners, so that the dressing adheres more reliably and for a longer time, so that the dressing does not need to be changed less frequently. All corners of the dressing may be rounded at a radius of 1cm, 2cm, 5cm, 10cm or more.

The outer shape of the dressing is defined by a contour edge, wherein the dressing may comprise one, two or more conformability cuts for enhancing conformability of the dressing to the lateral neck/collar area. A conformability cut may extend from the contour edge towards an interior portion of the dressing.

A contour edge is an outer edge of the dressing defining its overall outer shape or overall contour, excluding minute features or enveloping any such minute features.

A conformability cut, extending from the contour edge towards an interior of the dressing, may enhance conformability of the dressing, because it may facilitate bending at smaller radii of the contour edge in the area of the conformability cut. The portions of the dressing on both sides of the cut can move more freely with respect to each other, while remaining attached to the skin. The dressing therefore does not come off the skin easily in the area of the conformability cut. This improves its general adhesion to the skin and thereby increases the lifetime of the dressing on the skin, resulting in less frequent need for replacement.

Any conformability cut may extend from the contour edge, i.e. generally from the outer edge of the dressing, towards an interior of the dressing. A conformability cut may extend from the outer edge in a direction perpendicular to the outer edge. This direction may provide a most efficient protection against lifting, i.e. against the dressing coming off the skin, at the conformability cut, because bending is most harmful to adhesion when the bending axis is perpendicular to the edge of the self-adhesive dressing.

A conformability cut may extend 1mm or more, 3mm or more, 5mm or more from the outer edge of the dressing, towards an interior of the dressing. A conformability cut may be straight or curved.

Conformability cuts may be arranged around the entire contour edge of the dressing. Alternatively, conformability cuts may be arranged only in a portion of the contour edge in vicinity of a window in the dressing.

Generally, a dressing according to the invention may comprise a plurality of layers. One of the layers may be a layer of adhesive, e.g. of a pressure-sensitive adhesive. In other words, the dressing may comprise a layer of adhesive, e.g. of a pressure-sensitive adhesive. The adhesive may be suitable for adhering the dressing to the skin of a patient. A layer of adhesive may be continuous. Alternatively a layer of adhesive may be patterned. Patterned adhesive may be advantageous in that it may facilitate transmission of moisture and vapours from the skin to an outside of the dressing. This makes the wearing of the dressing more comfortable. A layer of adhesive may extend over the entire extension of the dressing. Alternatively, a layer of adhesive may extend over a first portion of the dressing, such that a second portion of the dressing remains free of adhesive. This may make arrangement of lumen under a portion of the dressing easier, because the lumen may not stick to adhesive-free portions and can then be pushed or pulled into a desirable position.

One of the layers of the dressing may be a layer of nonwoven material. In other words, the dressing may comprise a layer of nonwoven material. A layer of nonwoven material may be co-extensive with the dressing. Alternatively, a layer of nonwoven material may extend over a first portion of the dressing and leave a second portion free. A layer of nonwoven material may support or carry a layer of adhesive as described above.

One of the layers of the dressing may be a layer of conformable polymeric film. In other words, the dressing may comprise a layer of conformable polymeric film. The conformable polymeric film may be transparent. The polymeric film may extend over a window in the dressing, such as to allow visual inspection of skin or catheter through the window.

A layer of polymeric film may be adapted or selected such as to transmit moisture to the outside of the dressing. This may make the dressing more comfortable to wear.

A layer of polymeric film as described above may be co-extensive with the dressing. It may be co-extensive with a layer of nonwoven material. Alternatively, a layer of polymeric film may extend over a first portion of the dressing and leave a second portion free. A layer of polymeric film may support or carry a layer of adhesive as described above. A layer of polymeric film may be arranged between a layer of adhesive and a layer of nonwoven material.

Generally, in a dressing comprising a plurality of layers as described above, a conformability cut may extend, in a thickness direction, i.e. in a direction parallel to a surface normal of the dressing, through one layer of the dressing, through two layers, three layers, or generally through a plurality of layers, or through all layers of the dressing. In a specific embodiment of a dressing comprising a layer of polymeric film and a layer of nonwoven material, conformability cuts extend through the layer of nonwoven material, without extending through the layer of polymeric film. Generally, polymeric film in a dressing according to the present invention may be very thin and thereby highly conformable. Even if the layer of polymeric film does not comprise the cut, this layer is often conformable enough to not cause lifting of the dressing in the area of the cut when the dressing is bent in that area repeatedly.

The longest extension of the dressing along a straight line may define a length direction. Directions perpendicular to the length direction may define width directions. Length is extension in length direction, width is extension in width directions. The extension of the outer shape of the dressing in width directions may increase steadily in its length direction from a width of an end portion of the dressing up to a maximum width of the outer shape. The outer shape of such a dressing, in which the width increases steadily up to a maximum, may be particularly well suited to follow the limits of the lateral neck area of a patient without extending into adjacent body parts.

The outer shape of the skin surface of a lateral neck area of a human patient generally may be described as an elongated triangular, or as an elongated diverging, shape. It is contemplated that the outer shape has no axial symmetry. Hence a dressing having an elongated triangular, or alternatively an elongated diverging, axially-asymmetric outer shape may fit best to the skin surface in that lateral neck area and use the available skin surface most efficiently. The triangular outer shape of a dressing according to the invention may have no axial symmetry, e.g. with respect to a length direction of the dressing. Alternatively, the diverging outer shape of a dressing according to the invention may have no axial symmetry, e.g. no axial symmetry with respect to the length direction.

There is obviously one lateral neck area on the right-hand side of a patient, and a further lateral neck area on the left-hand side. An axially-asymmetric dressing according to the invention that is shaped to fit well on the right-hand lateral neck area will not fit well on the left-hand lateral neck area, and vice versa.

A dressing according to the invention may comprise a layer of adhesive. The layer of adhesive may render the dressing adhesive or self-adhesive. The surface area of the layer of adhesive may be 80 cm² or more. It may be 120 cm² or more, or 10 cm² or more. The adhesive may be a pressure-sensitive adhesive. The adhesive may be adapted for adhering to skin. The adhesive may be a skin-friendly adhesive, which may be easy to remove from skin without causing much pain.

The surface size of the layer of adhesive may determine a maximum size of a contact area between the adhesive and the patient's skin. Generally, the larger the contact area, the more force would be required to remove the dressing, and the more mechanical load could be carried by the dressing without failure. Larger contact areas therefore generally may provide for better adhesion and my support higher mechanical loads. A catheter securement dressing thus may need a minimum size of the contact area in order to support the weight of the catheter and its periphery, as well as ay pull forces on the catheter.

The outer shape of a dressing as described above may be chosen such that the dressing can be applied to the lateral neck area of more than 90% of human patients without the dressing extending beyond the lateral neck area of such patients.

Statistics may be available or can be generated that capture the size and shape of the lateral neck areas of a large number of potential patients. Using such data, a shape for a prefabricated dressing can be chosen that fits onto the lateral neck area of a large majority, e.g. more than 90%, of potential patients, without extending over adjacent body parts of those patients. This chosen shape may then be used to prefabricate dressings according to the invention in large numbers in an industrial process, hence cost-effectively.

The longest extension of the dressing along a straight line may be 5 centimeters or more. Alternatively, the longest extension of the dressing along a straight line may be 10, 12, 15 or 20 centimeters or more. These extensions of the dressing may ensure sufficient extension to cover wounds or secure a catheter reliably. Dressings for children generally need to be smaller in size, in order to not extend beyond the lateral neck area into other body parts, while dressings for adults can be as large as 15 cm or more in longest extension, without extending into other body parts.

A dressing as described above further comprises a window or a window portion. A window or a window portion is suitable for facilitating visual inspection of the patient's skin. The window may be arranged in an interior portion of the dressing, i.e. the window does not extend to an outer edge of the dressing.

A window may comprise a transparent film through which the skin can be visually inspected. The transparent film may be arranged in an opening of the dressing. The transparent film may carry an adhesive layer for attachment to the skin. Such a transparent film may help to provide protection against ingress of bacteria through the window, while still allowing visual inspection.

Alternatively, a window may comprise an opening in the dressing through which the skin underneath the window can be visually inspected.

A dressing according to the invention may comprise two or more windows of the types described above.

A dressing as described above may further comprise a first lumen securement cut or lumen securement slit, extending inwardly from the first long edge. A dressing may further comprise a second lumen securement cut or lumen securement slit, extending inwardly from the second long edge in a direction generally anti-parallel to the direction of the first securement cut. Generally, in this disclosure, anti-parallel directions are considered directions anti-parallel to each other within a +/-20° tolerance angle. Hence a dressing as described above may further comprise a second lumen securement cut or lumen securement slit, extending inwardly from the second long edge in a direction of an angle of between 160° and 200° relative to the direction of the first lumen securement cut. Specifically, it may be extending inwardly from the second long edge in a direction anti-parallel to the direction of the first lumen securement cut, i.e. in a direction of an angle of 180° relative to the direction of the first lumen securement cut.

A lumen securement cut may facilitate attaching and/or guiding and/or fixing tubes or lumen. One, two or more such securement cuts or securement slits may allow for easy and cost-effective attachment, guiding and/or fixation of tubes or lumen or the catheter itself, so that these are limited in their movement relative to the dressing. This may reduce mechanical stress on the catheter and thereby reduce the risk of catheter dislodgement. In a dressing comprising first and second lumen securement cuts, the lumen securement cuts may be arranged in directions antiparallel to each other and laterally spaced from each other by a spacing distance. The spacing distance may be between 0.5 cm and 10 cm, preferably between 1 cm and 6 cm, for example 2cm, 2.5 cm or 3 cm. A lumen securement cut may extend inwardly from the first or the second long edge, respectively, up to a line defined by the direction of greatest length of the dressing.

In a dressing comprising first and second lumen securement cuts extending inwardly from the first and the second long edge respectively, the lumen securement cuts may extend inwardly far enough to overlap with each other for a certain portion of their respective lengths. The overlap length may be defined as the length of the portion of the first lumen securement cut in which the first lumen securement cut runs next to, i.e. side by side with, a portion of the second lumen securement cut. The overlap length defines the width of a path which is available to attach, guide or fixate lumen or tubes via the lumen securement cuts. The overlap length may be between 1 cm and 5 cm. Specifically, it may be 1 cm, 2 cm or 3 cm.

Lumen securement cuts as described here may be advantageous in that they may facilitate securement of lumen on one-part dressings (e.g. dressings not using separate securement strips) without having to lift the dressing over its whole width, or without having to insert a lumen into a narrow space between skin and dressing, while the dressing is attached to skin. Having lumen securement cuts may make it obsolete to provide adhesive-free portions on the skin-facing surface of the dressing, which would facilitate pushing lumen between skin and dressing.

The invention will now be described in more detail with reference to the following Figures exemplifying particular embodiments of the invention:
- Fig. 1: Plan view of a first dressing according to the invention;
- Fig. 2: Sketch of the first dressing, applied on the lateral neck of a patient;
- Fig. 3: Perspective view of the first dressing, showing catheter and lumen, and
- Fig. 4: Plan view of a second dressing according to the invention.

Herein below various embodiments of the present invention are described and shown in the drawings wherein like elements are provided with the same reference numbers.

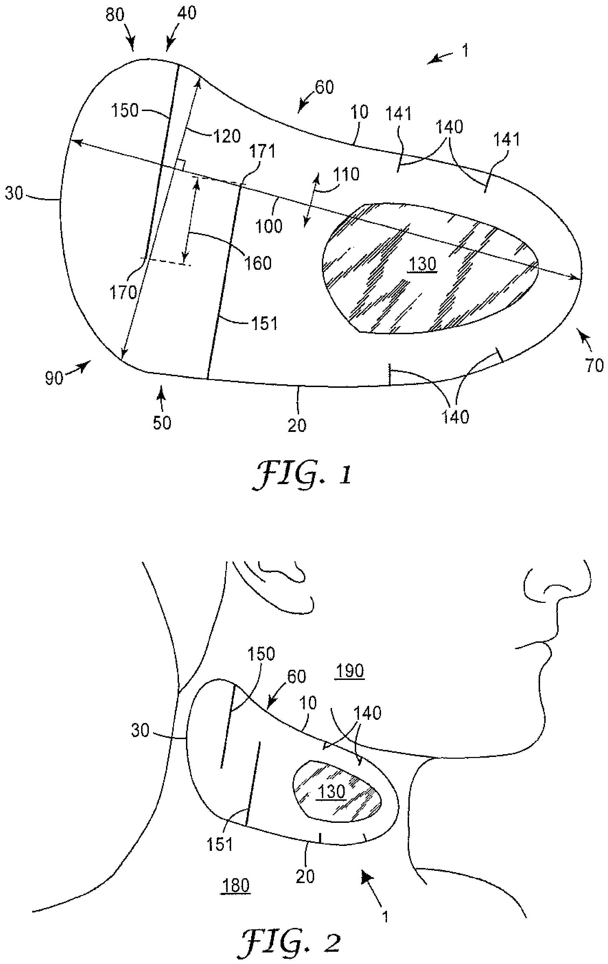

**Figure 1** is a plan view of a first dressing 1 according to the invention. The dressing 1 is a catheter securement dressing. It has an elongated triangular outer shape when flat. The outer shape is defined by a first long edge 10, a second long edge 20, opposite to the first long edge 10, and a short edge 30. The short edge 30 connects an end portion 40 of the first long edge 10 and an end portion 50 of the second long edge 20. The outer shape of the dressing 1 can also be described as elongated diverging, because the long edges 10, 20 diverge from each other.

The first long edge 10, the second long edge 20 and the third long edge 30 each define a respective curved line. The first long edge 10 defines a curved line which is shaped such that the dressing 1 comprises, in the plane of the dressing 1, a concave portion 60. The concave portion 60 is concave to the outside of the dressing 1.

The elongated triangular outer shape of the dressing 1 has no axial symmetry, because no axis can be identified relative to which the dressing 1 would be symmetric.

The dressing 1 is for application to the right-hand side lateral neck area of a human patient, as will be explained in the context of Figure 2. The concave portion 60 is shaped such as to fit to the contour of the jaw of the patient.

All three corners, namely a first corner 70, a second corner 80, and a third corner 90, are rounded. Rounded corners generally adhere better to a substrate than sharp corners.

The outer shape of the dressing 1 is defined by a contour edge, which is the outer edge forming the "circumference" of the dressing 1. The contour edge comprises the first long edge 10, the second long edge 20, the short edge 30 and the corner portions 70, 80, 90. The contour edge has the shape of a closed curve. The closed curve is smooth, i.e. it has no sharp steps, sharp corners or vertices, except for minor or small features stemming from imperfect manufacturing. For that reason the closed curve may be described as differentiable at any point of the closed curve, in a mathematical sense.

Line 100 indicates the direction of greatest length, or length direction, of the dressing 1. The extension of the dressing 1 in the direction 100 of greatest length is considered the length of the dressing 1 herein. The length of the dressing 1 is approximately 15 cm. Width directions 110 are directions perpendicular to the length direction 100. The direction of greatest width of the dressing 1 is indicated by line 120. The extension of the dressing 1 in the direction 120 of greatest width is considered the width of the dressing 1. It is about 8.8 cm.

The aspect ratio of the outer shape of the dressing 1 is defined as the greatest length in any direction, divided by the greatest width in a direction perpendicular to the direction of greatest length. Hence the first dressing 1 has an aspect ratio of length 100 divided by width 120, which is about 1.7.

The extension of the outer shape of the dressing 1 in width directions 110 increases steadily, when starting from the first corner 70 and advancing along the length direction 100. The width increases steadily up to the maximum width of the dressing at line 120.

Figure 1 can be considered a top view of the first dressing 1. The dressing 1 is a film dressing. It is flat and thin. Its thickness is less than 1mm. The underside of the dressing 1, not visible in Figure 1, is provided with a continuous layer of pressure-sensitive adhesive, by which the dressing 1 can be affixed to the skin of a patient. The layer of adhesive extends over the entire surface of the dressing 1.

An adhesive surface of this size can hold the weight of a catheter, when properly affixed to skin.

The dressing 1 comprises a visually transparent window 130, arranged in an interior portion of the dressing 1, i.e. surrounded completely by other portions of the dressing 1. The window 130 is formed by an opening in the layers of the dressing 1. The opening is provided with a transparent film. The window 130 is supposed to be applied over the catheter insertion point, where the catheter penetrates the skin. The window 130 allows this critical area of the patient's skin to be visually inspected without removing the dressing 1.

The dressing 1 comprises two types of cuts: Conformability cuts 140 and lumen securement cuts 150, 151. There are four conformability cuts 140, which help make the dressing 1 conformable to the skin of the patient. The conformability cuts 140 are straight cuts of about 5mm length. Two conformability cuts 140, those above the window 130 in Figure 1, extend from the first long edge 10 towards an interior portion of the dressing 1, i.e. they extend from the first long edge 10 inwardly. At their respective starting points 141, the conformability cuts 140 form a right angle with the first long edge 10. Since the first long edge 10 is curved, these conformability cuts 140 are not parallel to each other. Similarly, the two conformability cuts 140 below the window 130 in Figure 1, extend from the second long edge 20 towards an interior portion of the dressing 1, i.e. they extend from the second long edge 20 inwardly.

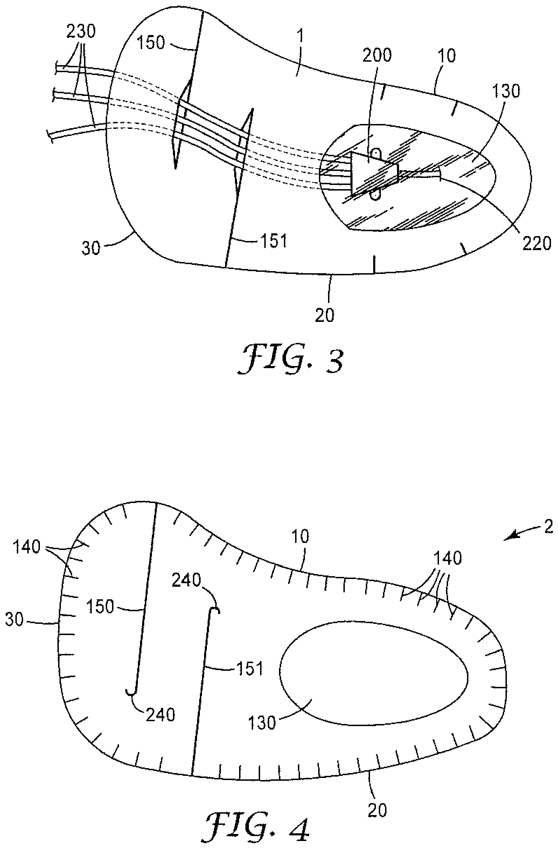

The dressing 1 comprises two lumen securement cuts 150, 151. These cuts are for attaching and guiding the tubes or lumen leading to or from the actual catheter, as is shown in Figure 3. The upper securement cut 150 starts at the first long edge 10 and extends inwardly. The lower securement cut 151 starts at the second long edge 20 and extends inwardly, in a direction anti-parallel to the upper securement cut 150. In order to avoid a risk of tearing the dressing 1 at the end of a securement cut 150, 151, it is contemplated to provide the securement cuts 150, 151 with u-shaped bends of small radius at their interior ends. The bend radius may be as small as 1-5 mm.

Both lumen securement cuts 150, 151 extend inwardly far enough to overlap with each other for a certain part of their respective lengths. The two cuts overlap where a section of the upper securement cut 150 and a section of the lower securement cut 151 actually run next to each other, i.e. side by side to each other. The amount of overlap is indicated by arrow 160.

Lumen 230 or tubes routed to or from the catheter insertion point 220 under window 130 towards the short edge 30 of the dressing 1 and further, can be secured by the securement cuts 150, 151 in the following manner: If a lumen 230 is routed such that it extends under the dressing 1 on one side (say the right-hand side in Figure 1) of the upper securement cut 150 and above the dressing 1 on the opposed side (the left-hand side in Figure 1) of the upper securement cut 150, the lumen 230 cannot move sideways, i.e. in a width direction 110 of the dressing 1, beyond an innermost end point 170 of the upper securement cut 150. If the lumen 230 is further routed such that it extends above the dressing 1 on one side (say the right-hand side in Figure 1) of the lower securement cut 151 and below the dressing 1 on the opposed side (the left-hand side in Figure 1) of the lower securement cut 151, the lumen 230 cannot move sideways, i.e. in a width direction 110 of the dressing 1, beyond an innermost end point 171 of the lower securement cut 151. In combination, and with an appropriate routing of the lumen 230, the lumen securement cuts 150, 151 confine lateral movement of the lumen 230 in directions parallel to the lumen securement cuts 150, 151 to the area of the overlap 160.

Movement of the lumen 230 and of the catheter 200 in directions along the lumen 230 are prevented by the adhesive layer on the skin-facing surface of the dressing 1 attaching to the catheter 200 and the lumen 230 in certain areas, and to the skin in other areas.

**Figure 2** is a perspective sketch of the dressing 1 described above, applied to the right-hand lateral neck area of a patient. A dressing for the left-hand side lateral neck area can be obtained by mirroring the shape of the dressing 1 for the right-hand side lateral neck area shown in Figure 2. The dressing 1 is arranged above the patient's shoulder area 180 and no portion of the dressing 1 extends into the shoulder area 180. The dressing 1 is arranged below the patient's jaw 190 and does not extend into the area of the jaw 190. The extension of the dressing 1 is slightly smaller than the vertical extension of the lateral neck between jaw area 190 and shoulder area 180. Thereby the dressing 1 does not extend beyond a joint into an adjacent body area, i.e. into the jaw area 190 or the shoulder area 180. On the one hand, this improves adhesion of the dressing 1 to the skin, because it is not designed to adhere to any skin areas, like jaw 190 or collar bone or shoulder area 180, to which it is hard to adhere for longer time, when the patient moves. On the other hand, this avoids discomfort for the patient, who might be impeded in his movements if the dressing 1 extended beyond a joint into an adjacent body area.

**Figure 3** is a perspective top view of the first dressing 1 of Figures 1 and 2, with a catheter 200 and associated lumen 230 shown as they could be applied on a patient's lateral neck area. The dressing 1 is arranged such that the catheter 200 and the catheter insertion point 220 are visible through the window 130 in order to allow monitoring. Three lumen 230 are routed from the outside to the catheter 200. They are arranged such as to go beneath or above the dressing 1 at the securement cuts 150, 151 as described above.

**Figure 4** is a plan view of a second catheter securement dressing 2 according to the invention. It is identical to the first dressing 1, except for the features described in the following.

The overall shape of the dressing 2 is slightly slimmer, i.e. its length/width ratio is greater than in the first dressing 1. Its aspect ratio is about 1.84. The shape of the window 130 is oval. Conformability cuts 140 are arranged around the entire contour edge of the dressing 2. They are spaced at about 5 mm from each other. The lumen securement cuts 150, 151 are not exactly parallel to each other. The lower securement cut 151 extends inwardly from the second long edge 20 in a direction generally anti-parallel to the direction of the upper securement cut 150. Specifically, the lower securement cut 151 extends inwardly from the second long edge 20 in a direction of an angle of about 183° relative to the direction of the upper securement cut 150.

Each of the lumen securement cuts 150, 151 has a U-shaped bend 240 at its interior end, to reduce the risk of tearing of the dressing 2 at the end of the lumen securement cuts 150, 151.

## Claims

1. Pre-fabricated self-adhesive flat catheter securement dressing (1) for application to a lateral neck area of a human patient, comprising a layer of pressure-sensitive adhesive for affixing the dressing to the patient's skin, and comprising a window (130) for facilitating visual inspection of the patient's skin,
**characterized in that** the dressing, in the plane of the dressing, has an elongated, generally triangular outer shape defined by a first long edge (10), a second long edge (20) opposite to the first long edge, and at least one short edge (30) connecting an end portion (40) of the first long edge and an end portion (50) of the second long edge, and wherein the triangular outer shape has no axial symmetry.

2. Dressing (1) according to claim 1, wherein the outer shape of the dressing is defined by a contour edge in the shape of a smooth closed curve, which is differentiable at any point of the curve.

3. Dressing according to any one of the preceding claims, wherein the outer shape of the dressing has an aspect ratio, defined as the greatest length (100) of the dressing in any direction divided by the greatest width (120) in a direction perpendicular to the direction of the greatest length, of 1.5 or more.

4. Dressing according to any one of the preceding claims, wherein the first long edge defines a curved line, the curved line being shaped such that the dressing comprises, in the plane of the dressing, a concave portion (60) being concave to the outside of the dressing.

5. Dressing according to any one of the preceding claims, wherein the elongated, generally triangular outer shape of dressing comprises a corner (70, 80, 90), wherein the corner is rounded at a radius of 1 cm or more.

6. Dressing according to any one of the preceding claims, wherein the elongated, generally triangular outer shape of the dressing comprises three corners (70, 80, 90), wherein each corner is rounded.

7. Dressing according to any one of the preceding claims, wherein the outer shape of the dressing is defined by a contour edge, wherein the dressing comprises one, two or more conformability cuts (140) for enhancing a conformability of the dressing to the lateral neck/collar area, the conformability cuts extending from the contour edge towards an interior portion of the dressing.

8. Dressing according to any one of the preceding claims, wherein longest extension of the dressing along a straight line defines a length direction (100), directions perpendicular to the length direction define width directions (110), and wherein the extension of the outer shape of the dressing in width directions increases steadily in its length direction up to a maximum width of the outer shape.

9. Dressing according to any one of the preceding claims, wherein the surface area of the layer of pressure-sensitive adhesive is 80 cm2 or more.

10. Dressing according to any one of the preceding claims, wherein the longest extension of the dressing along a straight line is 5 cm or more.

11. Dressing according to any one of the preceding claims, further comprising a first lumen securement cut (150), extending inwardly from the first long edge, and a second lumen securement cut (151), extending inwardly from the second long edge in a direction anti-parallel, within a +/- 20° angle, to the direction of the first securement cut, for attaching and guiding tubes or lumen (230).

## Patentansprüche

1. Vorgefertigter selbstklebender flacher Kathetersicherungsverband (1) zum Aufbringen auf einen seitlichen Nackenbereich eines menschlichen Patienten, umfassend eine Schicht aus druckempfindlichem Kleber zum Befestigen des Verbands an der Haut des Patienten und umfassend ein Fenster (130) zur Erleichterung der Sichtprüfung der Haut des Patienten,
**dadurch gekennzeichnet, dass** der Verband, in der Ebene des Verbands, eine längliche, im Allgemeinen dreieckige äußere Form aufweist, die durch eine erste lange Kante (10), eine der ersten langen Kante gegenüberliegende zweite lange Kante (20) und mindestens eine kurze Kante (30), die einen Endabschnitt (40) der ersten langen Kante und einen Endabschnitt (50) der zweiten langen Kante verbindet, definiert ist, und wobei die dreieckige äußere Form keine axiale Symmetrie aufweist.

2. Verband (1) nach Anspruch 1, wobei die äußere Form des Verbands durch eine Konturkante in Form einer glatten geschlossenen Kurve definiert ist, die an jedem Punkt der Kurve unterscheidbar ist.

3. Verband nach einem der vorstehenden Ansprüche, wobei die äußere Form des Verbands ein Seitenverhältnis, das als die größte Länge (100) des Verbands in jede Richtung geteilt durch die größte Breite (120) in einer Richtung senkrecht zur Richtung der größten Länge definiert ist, von 1,5 oder mehr aufweist.

4. Verband nach einem der vorstehenden Ansprüche, wobei die erste lange Kante eine gekrümmte Linie definiert, wobei die gekrümmte Linie derart geformt ist, dass der Verband in der Ebene des Verbands einen konkaven Abschnitt (60) umfasst, der zur Außenseite des Verbands konkav ist.

5. Verband nach einem der vorstehenden Ansprüche, wobei die längliche, im Allgemeinen dreieckige äußere Form des Verbands eine Ecke (70, 80, 90) umfasst, wobei die Ecke bei einem Radius von 1 cm oder mehr abgerundet ist.

6. Verband nach einem der vorstehenden Ansprüche, wobei die längliche, im Allgemeinen dreieckige äußere Form des Verbands drei Ecken (70, 80, 90) umfasst, wobei jede Ecke abgerundet ist.

7. Verband nach einem der vorstehenden Ansprüche, wobei die äußere Form des Verbandes durch eine Konturkante definiert ist, wobei der Verband einen, zwei oder mehr Anpassungsschnitte (140) zur Verbesserung einer Anpassungsfähigkeit des Verbands an den seitlichen Hals/Kragen-Bereich umfasst, wobei sich die Anpassungsschnitte von der Konturkante in Richtung eines inneren Abschnitts des Verbands erstrecken.

8. Verband nach einem der vorstehenden Ansprüche, wobei die längste Erstreckung des Verbands entlang einer geraden Linie eine Längsrichtung (100) definiert, Richtungen senkrecht zur Längsrichtung Breitenrichtungen (110) definieren und wobei die Erstreckung der äußeren Form des Verbands in Breitenrichtungen in seiner Längsrichtung bis zu einer maximalen Breite der äußeren Form stetig zunimmt.

9. Verband nach einem der vorstehenden Ansprüche, wobei der Oberflächenbereich der Schicht aus druckempfindlichem Kleber 80 cm2 oder mehr beträgt.

10. Verband nach einem der vorstehenden Ansprüche, wobei die längste Erstreckung des Verbands entlang einer geraden Linie 5 cm oder mehr beträgt.

11. Verband nach einem der vorstehenden Ansprüche, ferner umfassend einen ersten Lumensicherungsschnitt (150), der sich nach innen von der ersten langen Kante erstreckt, und einen zweiten Lumensicherungsschnitt (151), der sich nach innen von der zweiten langen Kante in einer Richtung antiparallel, in einem +/- 20°-Winkel, zu der Richtung des ersten Sicherungsschnitts, zum Anbringen und Führen von Schläuchen oder Lumen (230), erstreckt.

## Revendications

1. Pansement préfabriqué auto-adhésif plat d'immobilisation de cathéter (1) pour application à une région latérale de la nuque d'un patient humain, comprenant une couche d'adhésif sensible à la pression pour fixer le pansement à la peau du patient, et comprenant une fenêtre (130) pour faciliter une inspection visuelle de la peau du patient,
**caractérisé en ce que** le pansement, dans le plan du pansement, a une forme externe allongée, généralement triangulaire définie par un premier bord long (10), un deuxième bord long (20) opposé au premier bord long, et au moins un bord court (30) reliant une partie d'extrémité (40) du premier bord long et une partie d'extrémité (50) du deuxième bord long, et dans lequel la forme externe triangulaire ne présente aucune symétrie axiale.

2. Pansement (1) selon la revendication 1, dans lequel la forme externe du pansement est définie par un bord de contour sous la forme d'une courbe fermée régulière, qui est différentiable en n'importe quel point de la courbe.

3. Pansement selon l'une quelconque des revendications précédentes, dans lequel la forme externe du pansement a un rapport d'aspect, défini en tant que longueur la plus grande (100) du pansement dans n'importe quelle direction divisée par la largeur la plus grande (120) dans une direction perpendiculaire à la direction de la longueur la plus grande, de 1,5 ou plus.

4. Pansement selon l'une quelconque des revendications précédentes, dans lequel le premier bord long définit une ligne courbe, la ligne courbe étant profilée de telle sorte que le pansement comprend, dans le plan du pansement, une partie concave (60) étant concave vers l'extérieur du pansement.

5. Pansement selon l'une quelconque des revendications précédentes, dans lequel la forme externe allongée, généralement triangulaire du pansement comprend un coin (70, 80, 90), dans lequel le coin est arrondi à un rayon de 1 cm ou plus.

6. Pansement selon l'une quelconque des revendications précédentes, dans lequel la forme externe allongée, généralement triangulaire du pansement comprend trois coins (70, 80, 90), dans lequel chaque coin est arrondi.

7. Pansement selon l'une quelconque des revendications précédentes, dans lequel la forme externe du pansement est définie par un bord de contour, dans lequel le pansement comprend une, deux ou plus de deux découpes d'adaptabilité (140) pour améliorer une adaptabilité du pansement à la région latérale de la nuque/du cou, les découpes d'adaptabilité s'étendant à partir du bord de contour en direction d'une partie intérieure du pansement.

8. Pansement selon l'une quelconque des revendications précédentes, dans lequel l'extension la plus longue du pansement le long d'une ligne droite définit une direction en longueur (100), des directions perpendiculaires à la direction en longueur définissent des directions en largeur (110), et dans lequel l'extension de la forme externe du pansement dans les directions en largeur augmente régulièrement dans sa direction en longueur jusqu'à une largeur maximale de la forme externe.

9. Pansement selon l'une quelconque des revendications précédentes, dans lequel la superficie de la couche d'adhésif sensible à la pression est de 80 cm2 ou plus.

10. Pansement selon l'une quelconque des revendications précédentes, dans lequel l'extension la plus longue du pansement le long d'une ligne droite est de 5 cm ou plus.

11. Pansement selon l'une quelconque des revendications précédentes, comprenant en outre une première découpe d'immobilisation de lumière (150), s'étendant vers l'intérieur à partir du premier bord long, et une deuxième découpe d'immobilisation de lumière (151), s'étendant vers l'intérieur à partir du deuxième bord long dans une direction anti-parallèle, dans les limites d'un angle de +/- 20°, à la direction de la première découpe immobilisation, pour fixer et guider des tubes ou une lumière (230).
